# DEMANDE DE BREVET EUROPEEN

(11) **EP 1 543 817 A1**
(43) Date de publication de la demande: **22.06.2005**
(21) Numéro de dépôt: 04292664.2
(22) Date de dépôt: 10.11.2004
(51) Int. Cl.: A61K 7/075

(54) **Composition détergente contenant au moins un polymère comportant au moins un hétéroatome et au moins une huile**

(30) Priorité: 19.12.2003 FR 0351143
(71) Demandeur: L'OREAL, 75008 Paris (FR)
(72) Inventeur: Parris, Eric, 92110 Clichy (FR); Restle, Serge, 95390 Saint-Prix (FR)
(74) Mandataire: Le Blainvaux, Françoise

(57) **Abrégé**

La présente invention décrit une composition détergente comprenant, dans un milieu cosmétiquement acceptable au moins un polymère à hétéroatome et chaine grasse associé à au moins une huile, et au moins un tensioactif détergent anionique et/ou non ionique.

Cette composition peut être utilisée comme shampoing et permet d'obtenir de bonnes propriétés de conditionnement des cheveux.

## Description

La présente invention concerne une composition détergente contenant au moins un polymère comportant au moins un hétéroatome associé à au moins une huile, et un procédé de traitement cosmétique notamment capillaire mettant en oeuvre cette composition.

Dans le domaine de la cosmétique, on cherche notamment à améliorer le conditionnement des cheveux. Par conditionnement, on entend des propriétés de démêlage facile, de brillance, de douceur au toucher et de glissant et de lissage visuel et au toucher.

L'utilisation de polymère à chaîne grasse terminale et à motif récurrent contenant au moins un hétéroatome est connue en cosmétique, et plus particulièrement dans le domaine du maquillage comme cela est décrit dans les demandes de brevet FR2817743. Des compositions cosmétiques dont la phase grasse est gélifiée par de tels polymères ont été décrites la demande FR 2796270 qui décrit plus particulièrement des compositions solides de rouge à lèvre sous la forme d'un stick .

Le brevet US 5 783 657 illustre certains types des polymères qui peuvent entrer dans la composition des formules de l'invention. Néanmoins, ces documents ne décrivent pas de compositions cosmétiques détergentes.

La Demanderesse a trouvé de manière surprenante qu'en utilisant, dans des compositions détergentes de traitement capillaire, au moins un polymère à chaîne grasse terminale et/ou pendante et à motif récurrent contenant au moins un hétéroatome en association avec au moins une huile, il était possible d'obtenir un très bon effet de conditionnement des cheveux.

Sans être tenu par une quelconque théorie, il semblerait que dans ces conditions, on augmente significativement le dépôt d'huile sur les cheveux, d'où une efficacité accrue. Cette amélioration se fait cependant sans avoir un toucher chargé gras, ce qui est le cas habituellement lorsqu'on augmente la quantité d'huile.

Par ailleurs, cet effet de conditionnement peut être rémanent au rinçage.

L'invention a donc pour objet une composition détergente liquide comprenant, dans un milieu aqueux cosmétiquement acceptable, au moins un tensioactif détergent anionique ou non ionique, au moins une huile et au moins un polymère de masse moléculaire moyenne en poids inférieure ou égale à 1 000 000, comportant a) un squelette polymérique, ayant des motifs de répétition hydrocarbonés pourvus d'au moins un hétéroatome, et b) au moins une chaîne grasse pendante et/ou au moins une chaîne grasse terminale éventuellement fonctionnalisées, ayant de 6 à 120 atomes de carbone et étant liées à ces motifs hydrocarbonés, et de 70 à 95 % en poids d'eau par rapport au poids total de la composition.

L'invention a également pour objet une composition détergente liquide, comprenant dans un milieu aqueux cosmétiquement acceptable, au moins une huile, au moins un tensioactif détergent anionique ou non ionique, au moins un polymère de masse moléculaire moyenne en poids inférieure ou égale à 1 000 000, comportant a) un squelette polymérique, ayant des motifs de répétition hydrocarbonés pourvus d'au moins un hétéroatome, et b) au moins une chaîne grasse pendante et/ou au moins une chaîne grasse terminale éventuellement fonctionnalisées, ayant de 6 à 120 atomes de carbone et étant liées à ces motifs hydrocarbonés, et au moins un tensioactif amphotère.

Un objet supplémentaire de la présente invention est un procédé de traitement des matières kératiniques mettant en oeuvre une composition selon l'invention.

D'autres objets, caractéristiques, aspects et avantages de l'invention apparaîtront encore plus clairement à la lecture de la description et des divers exemples qui suivent.

Selon l'invention, la composition détergente comprend au moins un polymère défini ci-dessous associé à au moins une huile, et au moins un tensioactif détergent anionique ou non ionique.

Selon un mode particulier de l'invention, l'huile est, de préférence, pré-épaissie par le polymère ci-dessus (aussi appelé dans la suite de la description polymère structurant), c'est à dire qu'on mélange l'huile et le polymère avant l'introduction dans la composition.

Le rapport pondéral huile(s)/polymère(s) de l'invention est de préférence supérieur ou égal à 50/50, mieux encore supérieur ou égal à 60/40, plus préférentiellement compris entre 60/40 et 99/1 et encore plus préférentiellement compris entre 80/20 et 99/1.

Cette huile de préférence pré-épaissie est dispersée sous forme de particules dans la composition aqueuse. Les particules d'huile présentent de préférence une taille primaire moyenne en nombre comprise entre 1 et 100 µm, mieux encore entre 5 et 30 µm, et plus particulièrement de 10 à 20 µm.

Au sens de la présente invention, on entend par "taille primaire de particule", la dimension maximale qu'il est possible de mesurer entre deux points diamétralement opposés d'une particule individuelle. La taille peut être déterminée, par exemple, par microscopie électronique à transmission ou à partir de la mesure de la surface spécifique par la méthode BET ou bien par l'intermédiaire d'un granulomètre laser.

Le polymère structurant de la composition de l'invention est un solide non déformable à température ambiante (25°C) et pression atmosphérique (760 mm de Hg). Il est insoluble dans l'eau ou la phase aqueuse ; il est capable de structurer (épaissir) l'huile. En particulier, le polymère structurant ne cristallise pas et la structuration de la phase grasse liquide est due à des interactions hydrogène entre deux molécules de polymère et/ou entre les molécules du polymère et les molécules de la phase grasse liquide. De préférence, le polymère structurant n'a pas de groupe ionique.

Par "chaînes fonctionnalisées" au sens de l'invention, on entend une chaîne alkyle comportant un ou plusieurs groupes fonctionnels ou réactifs notamment choisis parmi les groupes amides, hydroxyle, éther, oxyalkylène ou polyoxyalkylène, halogène, dont les groupes fluorés ou perfluorés, ester, siloxane, polysiloxane. En outre, les atomes d'hydrogène d'une ou plusieurs chaînes grasses peuvent être substituées au moins partiellement par des atomes de fluor.

Selon l'invention, ces chaînes peuvent être liées directement au squelette polymérique ou via une fonction ester ou un groupement perfluoré.

Par "polymère", on entend au sens de l'invention un composé ayant au moins 2 motifs de répétition, et de préférence au moins 3 motifs de répétition, qui sont identiques.

Par "motifs de répétition hydrocarbonés", on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun de un à plusieurs hétéroatomes avantageusement non pendants et se trouvant dans le squelette polymérique. Ces hétéroatomes sont choisis parmi les atomes d'azote, de soufre, de phosphore et leurs associations, associés éventuellement à un ou plusieurs atomes d'oxygène. De préférence, les motifs comportent au moins un atome d'azote en particulier non pendant. Ces motifs comportent, en outre, avantageusement, un groupe carbonyle.

Les motifs à hétéroatome sont en particulier des motifs amide formant un squelette du type polyamide, des motifs carbamate et/ou urée formant un squelette polyuréthane, polyurée et/ou polyurée-uréthane. De préférence, ces motifs sont des motifs amide. Avantageusement, les chaînes pendantes sont liées directement à l'un au moins des hétéroatomes du squelette polymérique. Selon un mode de réalisation, le premier polymère comprend un squelette polyamide. En outre, les chaînes terminales sont liées au squelette polymérique, via un groupe de liaison qui peut être un groupe éther, amine, urée, uréthane, thioether, thioester, thiourée, thiouréthane ou une liaison simple.

Entre les motifs hydrocarbonés, le polymère peut comprendre des motifs siliconés ou des motifs oxyalkylénés.

En outre, le polymère de la composition de l'invention comprend avantageusement un nombre total de chaînes grasses qui représente de 40 à 98 % du nombre total des motifs à hétéroatome et des chaînes grasses, et mieux de 50 à 95 %. La nature et la proportion des motifs à hétéroatome est fonction de la nature de la phase grasse liquide et est en particulier similaire à la nature (polaire ou non) de la phase grasse liquide. Ainsi, plus les motifs à hétéroatome sont polaires et en proportion élevée dans le polymère, ce qui correspond à la présence de plusieurs hétéroatomes, plus le polymère a de l'affinité avec les huiles polaires. En revanche, plus les motifs à hétéroatome sont peu polaires voire apolaires ou en proportion faible, plus le polymère a de l'affinité avec les huiles apolaires.

Le polymère est de préférence un polyamide de masse moléculaire moyenne en poids inférieure à 1 000 000, comportant a) un squelette polymérique, ayant des motifs répétitifs amide, et b) éventuellement au moins une chaîne grasse pendante et/ou au moins une chaîne grasse terminale éventuellement fonctionnalisées, ayant de 6 à 120 atomes de carbone et étant liées à ces motifs amide.

De préférence, les chaînes grasses pendantes sont liées à l'un au moins des atomes d'azote des motifs amide du polymère.

En particulier, les chaînes grasses de ce polyamide représentent de 40 à 98 % du nombre total des motifs à hétéroatome du polymère, et mieux de 50 à 95 %.

Avantageusement, le polymère structurant et en particulier le polyamide, de la composition selon l'invention présente une masse moléculaire moyenne en poids inférieure à 1 000 000 et mieux à 500 000. De préférence, cette masse moléculaire est inférieure ou égale à 100 000 (notamment allant de 1000 à 100 000), en particulier inférieure ou égale à 50 000 (notamment allant de 1000 à 50 000), et plus particulièrement allant de 1000 à 30 000, de préférence de 2000 à 20 000, et mieux de 2000 à 10 000.

Comme polymères structurant préférés utilisables dans l'invention, on peut citer les polyamides, de préférence ramifiés par des chaînes grasses pendantes et/ou des chaînes grasses terminales contenant de 6 à 120 atomes de carbone, en particulier ayant de 12 à 120 atomes de carbone et notamment de 12 à 68 atomes de carbone, les chaînes grasses terminales étant liées au squelette polyamide par des groupes de liaison notamment ester.

Ces polymères sont de préférence des polymères résultant d'une polycondensation entre un diacide carboxylique à au moins 32 atomes de carbone (ayant notamment de 32 à 44 atomes de carbone) et une diamine ayant au moins 2 atomes de carbone (notamment de 2 à 36 atomes de carbone). Le diacide est de préférence un dimère d'acide gras ayant au moins 16 atomes de carbone comme l'acide oléique, linoléique ou linolénique. La diamine est de préférence l'éthylène diamine, l'hexylène diamine, l'hexaméthylène diamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple 18 atomes de carbone.

Ces polymères sont plus spécialement ceux décrits dans le document US-A-5783657 de la société Union Camp. Chacun de ces polymères satisfait notamment à la formule (I) suivante: 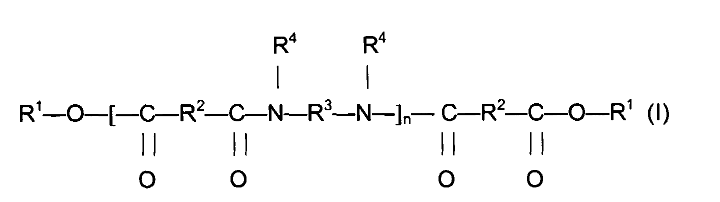 dans laquelle n désigne un nombre entier de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone par exemple de 4 à 24 atomes de carbone; R² représente à chaque occurrence indépendamment un groupe hydrocarboné en C₄ à C₄₂ à condition que 50 % des groupes R² représentent un groupe hydrocarboné en C₃₀ à C₄₂ ; R³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

En particulier, les groupes ester de la formule (I), qui font partie des chaînes grasses terminales et/ou pendantes au sens de l'invention, représentent de 15 à 40 % du nombre total des groupes ester et amide et mieux de 20 à 35 %. De plus, n représente avantageusement un nombre entier allant de 1 à 10 par exemple, de 1 à 5 et mieux supérieur à 2. De préférence, R¹ est un groupe alkyle en C₁₂ à C₂₂ et de préférence en C₁₆ à C₂₂. Avantageusement, R² peut être un groupe hydrocarboné (alkylène) en C₁₀ à C₄₂. De préférence, 50 % au moins et mieux 75 % des R² sont des groupes ayant de 30 à 42 atomes de carbone. Les autres R² sont des groupes hydrogénés en C₄ à C₁₉ et même en C₄ à C₁₂. De préférence, R³ représente un groupe hydrocarboné en C₂ à C₃₆ ou un groupe polyoxyalkyléné et R⁴ représente un atome d'hydrogène. De préférence, R³ représente un groupe hydrocarboné en C₂ à C₁₂.

Les groupes hydrocarbonés peuvent être des groupes linéaires, cycliques ou ramifiés, saturés ou insaturés. Par ailleurs, les groupes alkyle et alkényle peuvent être des groupes linéaires ou ramifiés.

Selon l'invention, la structuration (ou épaississement) de la phase grasse liquide est obtenue à l'aide d'un ou plusieurs polymères selon l'iinvention en particulier de formule (I). En général, les polymères de formule (I) se présentent sous forme de mélanges de polymères, ces mélanges pouvant en outre contenir un produit de synthèse correspondant à un composé de formule I) où n vaut 0, c'est-à-dire un diester.

A titre d'exemple de polymères structurant utilisables dans la composition selon l'invention, on peut citer les produits commerciaux fabriqués ou vendus par la société Arizona Chemical sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94°C. Ces produits commerciaux sont un mélange de copolymère d'un diacide en C₃₆ condensé sur l'éthylène diamine, de masse moléculaire moyenne d'environ 6000. Les groupes ester terminaux résultent de l'estérification des terminaisons d'acide restantes par l'alcool cétylique, stéarylique ou leurs mélanges (appelé aussi alcool cétylstéarylique).

Comme polymère structurant utilisable dans l'invention, on peut encore citer les résines polyamides résultant de la condensation d'un acide di-carboxylique aliphatique et d'un diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp. (Versamid 930, 744 ou 1655) ou par la société Olin Mathieson Chemical Corp., sous la marque Onamid® notamment Onamid S ou C. Ces résines ont une masse moléculaire moyenne en poids allant de 6000 à 9000. Pour plus d'information sur ces polyamides, on peut se référer aux documents US-A-3645705 et US-A-3148125. Plus spécialement, on utilise les Versamid® 930 ou 744.

On peut aussi utiliser les polyamides fabriqués ou vendus par la société Arizona Chemical sous les références Uni-Rez® (2658, 2931, 2970, 2621, 2613, 2624, 2665, 1554, 2623, 2662) et le produit vendu sous la référence Macromelt 6212 de la société Henkel. Pour plus d'information sur ces polyamides, on peut se référer au document US-A-5500209.

Il est aussi possible d'utiliser des résines de polyamides comme celles décrites dans les brevets US-A-5783657 et US-A-5998570.

Les polymères structurant de la composition de l'invention ont avantageusement une température de ramollissement supérieure à 65°C et mieux supérieure à 70°C et pouvant aller jusqu'à 190°C. De préférence, ils présentent une température de ramollissement inférieur à 150°C, par exemple allant de 70 à 140°C et mieux allant de 80 à 130°C et mieux encore de 80 à 105°C. Ces polymères sont en particulier des polymères non cireux. Le bas point de fusion des polymères structurant de l'invention facilite leur mise en oeuvre et limite la dégradation de la phase grasse liquide, contrairement à des polymères ou composés de point de ramollissement plus élevé.

De préférence, les polymères de la composition selon l'invention sont ceux répondant à la formule (I). Ces polymères présentent du fait de leur (s) chaîne (s) grasse (s), une bonne solubilité dans les huiles et donc conduisent à des compositions macroscopiquement homogènes même avec un taux élevé (au moins 25%) de polymère, contrairement à des polymères exempts de chaîne grasse.

Dans toute la description, les valeurs de température de ramollissement ou de fusion peuvent être déterminées par la méthode D.S.C ("Differential Scanning Calorimetry") ; la température de ramollissement ou de fusion correspond alors au pic de fusion et la montée en température est de 5 ou 10°C/min.

L'épaississement de la phase grasse est modulable selon la nature du ou des polymères et leurs concentrations et peut être telle que l'on obtienne de préférence une viscosité allant de 1000 à 250.000 cps et de préférence de 10.000 à 50.000 cps, à 25°C mesurée avec un appareil RHEOMAT 180 avec un taux de cisaillement de 100s⁻¹.

Le polymère tel que défini ci-dessus est de préférence présent en une quantité comprise entre 0,005 et 20 % en poids, mieux encore en une quantité comprise entre 0,05 et 10 % en poids, et encore plus préférentiellement en une quantité comprise entre 0,05 et 5 % en poids et plus particulièrement entre 0,1 et 2% en poids par rapport au poids de la composition.

Par huile, au sens de la demande, on entend un corps gras liquide insoluble dans l'eau à température ambiante (25°C) et pression atmosphérique (760 mm de Hg). La phase huileuse peut être composée d'une ou plusieurs huiles, compatibles entre elles.

Par insoluble dans l'eau, on entend au sens de la présente invention, une substance qui présente une solubilité dan l'eau pure inférieure à 1 % à 25 °C et sous pression atmosphérique.

Les huiles utilisées dans la présente invention présente une viscosité dynamique à 25 °C, inférieure à 1 Pa.s (1000 cps), de préférence comprise entre 10⁻³ et 0,1 Pa.s (1 et 100 cps). La viscosité dynamique est mesurée à 25 °C avec un taux de cisaillement de 100 s⁻¹, par exemple, avec l'appareil référencé RM 180 Rheomat de la société METTLER.

Les huiles pouvant être utilisées dans la présente invention sont choisies de préférence, parmi les huiles végétales, les huiles minérales, les huiles de synthèse, et les esters d'acide gras.

Parmi les huiles végétales pouvant être utilisées dans la présente invention, on peut notamment citer l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot et l'huile de calophyllum.

Des exemples d'huiles minérales sont l'huile de paraffine et l'huile de vaseline.

Les huiles de synthèse peuvent notamment être choisies parmi les polydécènes, le squalane, les poly(alpha-oléfines) comme l'isododécane ou l'isohexadécane, les huiles végétales transestérifiées et les huiles fluorées.

On peut également utiliser des esters d'acide gras, tels que par exemple, les composés de formule RₐCOOR_{b} dans laquelle Rₐ représente le reste d'un acide gras supérieur comportant de 5 à 29 atomes de carbone et R_{b} représente une chaîne hydrocarbonée contenant de 3 à 30 atomes de carbone, telles que l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyldodécyle.

Les huiles particulièrement préférées dans le cadre de la présente invention sont l'huile d'avocat, l'huile de ricin, l'huile d'olive, le polydécène hydrogéné, le myristate d'isopropyle, l'isononanoate d'isononyle, une paraffine liquide

La ou les huile(s) telle(s) que définie(s) ci-dessus est ou sont de préférence présente(s) en une quantité comprise entre 0,01 et 30 % en poids, encore plus préférentiellement en une quantité comprise entre 0,1 et 15 % en poids et plus particulièrement de 0,1 à 10% en poids par rapport au poids de la composition et notamment de 0,5 à 5% en poids.

A titre de tensioactifs détergents anioniques utilisables, seuls ou en mélanges, dans le cadre de la présente invention, on peut citer notamment les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les sulfates d'alkyle, les alkyléther-sulfates, les alkylamidoéthersulfates, les alkylarylpolyéthersulfates, les monoglycéride-sulfates ; les alkylsulfonates, les phosphates d'alkyle, les alkylamidesulfonates, les alkylarylsulfonates, les alpha-oléfine-sulfonates, les paraffine-sulfonates ; les sulfosuccinates d'alkyle, les alkyléthersulfosuccinates, les alkylamidesulfosuccinates ; les sulfoacétates d'alkyle ; les acylsarcosinates ; et les acylglutamates, les groupes alkyle ou acyle de tous ces composés comportant de 6 à 24 atomes de carbone et le groupe aryle désignant de préférence un groupe phényle ou benzyle. On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les sulfosuccinamates d'alkyle, les iséthionates d'acyle et les N-acyltaurates, le groupe alkyle ou acyle de tous ces composés comportant de 12 à 20 atomes de carbone. Parmi les tensioactifs anioniques encore utilisables, on peut également citer les lactylates d'acyle dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Parmi les tensioactifs anioniques cités ci-dessus, on préfère utiliser selon l'invention les sels, en particulier de sodium, de magnésium ou d'ammonium, de sulfates d'alkyle ; d'alkyléther-sulfates, comme le lauryléthersulfate de sodium, de préférence à 2 ou 3 moles d'oxyde d'éthylène ; d'alkyléthercarboxylates ; et leurs mélanges, les groupes alkyle comportant généralement de 6 à 24 atomes de carbone, et de préférence de 8 à 16 atomes de carbone.

Les agents tensioactifs non-ioniques détergents utilisables dans la composition selon l'invention, sont des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)aminopropylmorpholine ; et leurs mélanges.

Parmi les tensioactifs non-ioniques cités ci-dessus, on utilise de préférence les alkyl(C₆-C₂₄)polyglycosides et plus particulièrement les alkyl(C₈-C₁₆)polyglycosides.

Le ou les tensioactifs anioniques ou non ioniques sont généralement présents en une quantité comprise entre 3 et 50 % en poids, de préférence entre 4 et 30 % en poids, encore plus préférentiellement entre 5 et 20 % en poids par rapport au poids total de la composition détergente.

La composition détergente selon l'invention peut comprendre en outre un ou plusieurs tensioactifs amphotères et éventuellement des tensioactifs non détergents en particulier non ioniques.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant, au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes ; et leurs mélanges.

Parmi les dérivés d'amines, on peut citer les produits commercialisés sous la dénomination MIRANOL®, tels que décrits dans les brevets US-2 528 378 et US-2 781 354 et classés dans le dictionnaire CTFA, 3^{ème} édition, 1982, sous les dénominations Amphocarboxy-glycinate et Amphocarboxypropionate de structures respectives (2) et (3) :

R₂-CONHCH₂CH₂-N⁺(R₃)(R₄)(CH₂COO⁻) (2)

dans laquelle :
R₂ représente un groupe alkyle dérivé d'un acide R₂-COOH présent dans l'huile de coprah hydrolysée, un groupe heptyle, nonyle ou undécyle,
R₃ représente un groupe bêta-hydroxyéthyle, et
R₄ représente un groupe carboxyméthyle ;
et

R_{2'}-CONHCH₂CH₂-N(B)(C) (3)

dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
R_{2'} représente un groupe alkyle d'un acide R₂-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL® C2M concentré par la société RHODIA.

Parmi les tensioactifs amphotères, on utilise de préférence les alkyl(C₈-C₂₀)bétaïnes, les alkyl(C₈-C₂₀)amidoalkyl(C₆-C₈)bétaïnes, les alkylamphodiacétates et leurs mélanges.

Les tensioactifs amphotères sont généralement présents en une quantité comprise entre 0,1 et 20 % en poids, de préférence entre 0,5 et 15 % en poids, encore plus préférentiellement entre 1 et 10 % en poids par rapport au poids total de la composition détergente.

La composition selon l'invention peut notamment comprendre en outre un ou plusieurs polymères cationiques. Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont ceux décrits dans les brevets français n^{os} 2 505 348 et 2 542 997. Parmi ces polymères, on peut citer :
(1) les homopolymères ou copolymères dérivés d'esters ou d'amides de l'acide acrylique ou méthacrylique ;
(2) les dérivés d'éthers de cellulose comportant des groupements ammonium quaternaire décrits dans le brevet français 1 492 597 ;
(3) les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl- celluloses greffées notamment avec un sel de méthacryloyoxyléthyl-triméthylammonium, de méthacrylamidopropyl-triméthylammonium ou de diméthyldiallylammonium ;
(4) les polysaccharides cationiques décrits plus particulièrement dans les brevets US 3 589 578 et 4 031 307 tels que les gommes de guar contenant des groupements cationiques trialkylammonium ;
(5) les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361 ;
(6) les polyaminoamides solubles dans l'eau, tels que ceux notamment décrits dans les brevets français 2 252 840 et 2 368 508 ;
(7) les dérivés de polyaminoamides, par exemple, les polymères acide adipique/diakylaminohydroxyalkyldialkylène-triamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.
(8) les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347 ;
(9) les cyclopolymères d'alkyldiallylamine ou de dialkyldiallylammo-nium tels que l'homopolymère de chlorure de diméthyldiallyl-ammonium et les copolymères de chlorure de diallyldiméthylammonium et d'acrylamide ;
(10) les polymères de diammonium quaternaire présentant une masse moléculaire moyenne en nombre généralement comprise entre 1000 et 100000, tels que, ceux décrits, par exemple, dans les brevets français 2 320 330, 2 270 846, 2 316 271, 2 336 434 et 2 413 907 et les brevets US 2 273 780, 2 375 853, 2 388 614, 2 454 547, 3 206.462, 2 261 002, 2 271 378, 3.874.870, 4.001.432, 3 929 990, 3 966 904, 4 005 193, 4 025 617, 4 025 627, 4 025 653, 4 026 945 et 4 027 020 ;
(11) les polymères de polyammonium quaternaire tels que ceux notamment décrits dans la demande de brevet EP-A-122 324 ;
(12) les polymères quaternaires de vinylpyrrolidone et de vinylimidazole tels que, par exemple, les produits commercialisés sous les dénominations Luviquat® FC 905, FC 550 et FC 370 par la société B.A.S.F ;
(13) les polyamines comme le Polyquart® H vendu par HENKEL, référencées sous le nom de « POLYETHYLENEGLYCOL (15) TALLOW POLYAMINE » dans le dictionnaire CTFA ;
(14) les polymères réticulés de sels de méthacryloyloxyalkyl(C₁-C₄) trialkyl(C₁-C₄)ammonium tels que ceux commercialisés sous le nom de SALCARE® SC 92, SALCARE® SC 95 et SALCARE® SC 96 par la Société ALLIED COLLOIDS ; et
leurs mélanges.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, convenant dans l'invention, on peut utiliser de préférence les dérivés d'éther de cellulose quaternaires, les gommes de guar cationiques, les cyclopolymères cationiques, les polymères quaternaires de vinylpyrrolidone et de vinylimidazole, et leurs mélanges.

Le ou les polymères cationiques sont généralement présents à des concentrations allant de 0,01 à 20%, de préférence de 0,05 à 10% et plus particulièrement de 0,1 à 5 % en poids par rapport au poids total de la composition.

Par milieu cosmétiquement acceptable, on entend un milieu compatible avec les matières kératiniques, notamment les cheveux, mais aussi d'odeur, d'aspect et de toucher agréables.

Le milieu cosmétiquement acceptable comprend l'eau ou un mélange d'eau et d'un solvant cosmétiquement acceptable choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

De préférence, la composition comprend de 70 à 95 % en poids d'eau par rapport au poids total de la composition.

Les compositions de lavage selon l'invention présentent un pH final généralement compris entre 3 et 10. De préférence, ce pH est compris entre 4,5 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants (associatifs ou non associatifs). On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères d'acide acrylique tels que les copolymères d'acide acrylique et d'acrylates d'alkyle en C₁₀-C₃₀ réticulés. Ces agents régulateurs de viscosité sont utilisés dans les composition selon l'invention dans des proportions pouvant aller jusqu'à 10 % en poids par rapport au poids total de la composition.

Les compositions conformes à l'invention peuvent également contenir de préférence jusqu'à 5 % d'agents nacrants ou opacifiants bien connus dans l'état de la technique tels que par exemple les alcools gras supérieurs à C16, les dérivés acylés à chaîne grasse tels que les monostéarates ou distéarates d'éthylène glycol ou de polyéthylèneglycol, les éthers à chaînes grasses (C10-C30) tels que par exemple le distéaryléther ou le 1-(hexadécyloxy)-2-octadécanol.

Les compositions conformes à l'invention peuvent éventuellement contenir en outre au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les tensioactifs cationiques, les protéines, les hydrolysats de protéines, les céramides, les pseudocéramides, les acides gras à chaînes linéaires ou ramifiées en C₁₂-C₄₀ tels que l'acide méthyl-18 eicosanoique, les hydroxyacides, les vitamines telles que notamment les vitamines E, C, B, les provitamines tels que le panthénol, les silicones, les huiles animales, minérales ou de synthèse, les polymères anioniques, cationiques, non-ioniques amphotères ou zwitteroniques différents des polymères comportant un hétéroatome selon l'invention, les filtres UV, les parfums, les colorants, les épaississants naturels ou synthétiques, les alcools gras en C₁₂-C₃₀, les agents nacrants, les conservateurs, les agents de stabilisation de pH, les agents antimicrobiens, les agents anti-pelliculaires ou anti-séborrhéiques, les agents anti-oxydants ou réducteurs, les agents acides ou alcalins et leurs mélanges et tout autre additif classiquement utilisé dans le domaine cosmétique qui n'affecte pas les propriétés des composition selon l'invention.

Les composition de lavage selon l'invention peuvent bien entendu contenir en outre tous les adjuvants usuels rencontrés dans le domaine des shampooings, comme par exemple des parfums, des agents conservateurs, des sequestrants, des adoucissants, des colorants, des agents hydratants, des agents anti-pelliculaires ou anti-séborrhéiques, des agents antichute des cheveux, et autres.

Bien entendu, l'homme de l'art veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Ces compositions peuvent se présenter sous la forme de liquides, éventuellement épaissis, de crèmes ou de gel et elles conviennent principalement au lavage des cheveux.

Les compositions selon l'invention se présentent de préférence sous la forme d'une émulsion huile-dans-eau.

Les compositions peuvent être conditionnées sous diverses formes notamment dans des flacons, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir une mousse pour le traitement des cheveux.

La présente invention concerne également un procédé de traitement cosmétique des matières kératiniques qui consiste à appliquer une quantité efficace d'une composition telle que décrite ci-dessus, sur les matières kératiniques, à effectuer un rinçage après un éventuel temps de pose.

Les compositions conformes à l'invention peuvent être utilisées en tant que shampoings ou gel douche et de préférence comme shampooing pour les cheveux.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

Les exemples suivants illustrent la présente invention.

### Exemple 1

On a préparé des mélanges de polymères avec les huiles indiqués dans le tableau ci-dessous dans les rapports indiqués ci-dessous :

| Huile | Polymère | Rapport huile/polymère | Viscosité |
|---|---|---|---|
| Huile de ricin | Copolymère d'un diacide en C₃₆ condensé sur l'éthylène diamine (UNICLEAR 100VG de ARIZONA CHEMICAL) | 90/10 | 9500 cps |
| Isononanoate d'isonyle | | 90/10 | 14 500 cps |

### Exemple 2

Le shampoing suivant a été préparé avec l'huile épaissie décrite à l'exemple 1. Les proportions sont indiquées en % en poids.

| Composition | Ex 2 |
|---|---|
| Lauryléthersulfate de sodium à 70 % | 14 g MA |
| Cocobétaïne à 30 % | 2,8 g MA |
| Cocamide MIPA⁽¹⁾ | 1,5 g MA |
| Carbomer⁽²⁾ | 0,2 g |
| Isononanoate d'isononyle | 2,83 g |
| UNICLEAR 100VG | 0,17 g MA |
| Hydroxystéaryl céthyl éther et alcool cétylique | 2,5 g |
| Agent de pH qs | PH 7 |
| Conservateur qs | |
| Eau qsp | 100g |

| | |
|---|---|
| MA : Matière Active ⁽¹⁾ vendu sous la marque commerciale Empilan CIS par la société HUNSTSMAN | |
| ⁽²⁾ vendu sous la marque commerciale Carbopol 980 par la société Noveon | |

### MA : Matière Active

Cette composition appliquée sur cheveux naturels leur confère après 3 minutes de temps de pose et un rinçage, un excellent effet conditionneur.

## Revendications

1. Composition détergente liquide, comprenant dans un milieu aqueux cosmétiquement acceptable, au moins une huile, au moins un tensioactif détergent anionique ou non ionique, au moins un polymère de masse moléculaire moyenne en poids inférieure ou égale à 1 000 000, comportant a) un squelette polymérique, ayant des motifs de répétition hydrocarbonés pourvus d'au moins un hétéroatome, et b) au moins une chaîne grasse pendante et/ou au moins une chaîne grasse terminale éventuellement fonctionnalisées, ayant de 6 à 120 atomes de carbone et étant liées à ces motifs hydrocarbonés, et de 70 à 95 % en poids d'eau par rapport au poids total de la composition.

2. Composition détergente liquide, comprenant dans un milieu aqueux cosmétiquement acceptable, au moins une huile, au moins un tensioactif détergent anionique ou non ionique, au moins un polymère de masse moléculaire moyenne en poids inférieure ou égale à 1 000 000, comportant a) un squelette polymérique, ayant des motifs de répétition hydrocarbonés pourvus d'au moins un hétéroatome, et b) au moins une chaîne grasse pendante et/ou au moins une chaîne grasse terminale éventuellement fonctionnalisées, ayant de 6 à 120 atomes de carbone et étant liées à ces motifs hydrocarbonés, et au moins un tensioactif amphotère.

3. Composition selon la revendication 1 ou 2, **caractérisée par le fait que** la masse moléculaire moyenne du premier polymère est inférieure ou égale à 500 000 et mieux inférieure ou égale à 100 000.

4. Composition selon l'une quelconque des revendications précédentes, **caractérisée par le fait que** les motifs à hétéroatome du polymère comportent au moins un atome d'azote.

5. Composition selon l'une des revendications précédentes, **caractérisée par le fait que** les motifs à hétéroatome sont des groupes amides.

6. Composition selon l'une des revendications précédentes, **caractérisée par le fait que** les chaînes grasses représentent de 40 à 98 % du nombre total des motifs à hétéroatome et des chaînes grasses.

7. Composition selon l'une des revendications précédentes, **caractérisée par le fait que** les chaînes grasses représentent de 50 à 95 % du nombre total des motifs à hétéroatome et des chaînes grasses.

8. Composition selon l'une des revendications précédentes, **caractérisée par le fait que** les chaînes grasses pendantes sont liées directement à l'un au moins desdits hétéroatomes.

9. Composition selon l'une des revendications 3 à 7, **caractérisée par le fait que** les chaînes grasses pendantes sont liées directement à l'un au moins des atomes d'azote des motifs amide.

10. Composition selon l'une des revendications précédentes, **caractérisée par le fait que** la masse moléculaire moyenne en poids va de 1 000 à 30 000, et mieux de 2 000 à 10 000.

11. Composition selon l'une des revendications précédentes, **caractérisée par le fait que** les chaînes grasses terminales sont liées au squelette par des groupes de liaison.

12. Composition selon la revendication 10, **caractérisée par le fait que** les groupes de liaison sont des groupes ester.

13. Composition selon l'une des revendications précédentes, **caractérisée par le fait que** les chaînes grasses ont de 12 à 68 atomes de carbone.

14. Composition selon l'une des revendications précédentes, **caractérisée par le fait que** le polymère est choisi parmi les polymères de formule (I) suivante et leurs mélanges : 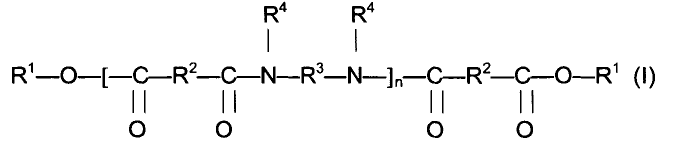 dans laquelle n désigne un nombre de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone ; R² représente à chaque occurrence indépendamment un groupe hydrocarboné en C₄ à C₄₂ à condition que 50 % des groupes R² représentent un groupe hydrocarboné en C₃₀ à C₄₂; R³ représente à chaque occurrence indépendamment un groupe organique pourvus d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

15. Composition selon la revendication précédente, **caractérisée par le fait que** R¹ est un groupe alkyle en C₁₂ à C₂₂.

16. Composition selon l'une des revendications 14 ou 15, **caractérisée par le fait que** R² est un groupe ayant de 30 à 42 atomes de carbone.

17. Composition selon l'une quelconque des revendications précédentes, **caractérisée en ce que** le polymère est présent en une quantité comprise entre 0,005 et 20 % en poids, de préférence entre 0,05 et 10 % en poids par rapport au poids total de la composition.

18. Composition selon l'une quelconque des revendications précédentes, **caractérisée en ce que** l'huile est choisie parmi les huiles végétales, les huiles minérales, les huiles de synthèse, et les esters d'acides gras.

19. Composition selon la revendication 18, **caractérisée en ce que** l'huile est choisie parmi l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum ; l'huile de paraffine et l'huile de vaseline ; les polydécènes, le squalane, les poly(alpha-oléfines) comme l'isododécane ou l'isohexadécane, les huiles végétales transestérifiées et les huiles fluorées ; les composés de formule RₐCOOR_{b} dans laquelle Rₐ représente le reste d'un acide gras supérieur comportant de 5 à 29 atomes de carbone et R_{b} représente une chaîne hydrocarbonée contenant de 3 à 30 atomes de carbone.

20. Composition selon la revendication 19, **caractérisée en ce que** l'huile est choisie parmi l'huile d'avocat, l'huile de ricin, l'huile d'olive, l'isohexadécane, le polydécène , le myristate d'isopropyle, l'isononanoate d'isononyle, une paraffine liquide.

21. Composition selon l'une quelconque des revendications précédentes, **caractérisée en ce que** la ou le(s) huile(s) est ou sont présente(s) en une quantité comprise entre 0,01 et 30 % en poids, de préférence entre 0,1 et 15 % en poids par rapport au poids total de la composition.

22. Composition selon l'une des revendications précédentes, **caractérisée en ce que** l'huile est pré-épaissie par le polymère comportant un hétéroatome.

23. Composition selon l'une des revendications précédentes, **caractérisée en ce que** le rapport pondéral huile(s)/polymère comportant un hétéroatome est supérieur ou égal à 50/50.

24. Composition selon la revendication 23, **caractérisée en ce que** le rapport pondéral huile(s)/polymère comportant un hétéroatome est supérieur ou égal à 60/40, de préférence compris entre 60/40 et 99/1.

25. Composition selon l'une quelconque des revendications précédentes, **caractérisée en ce que** l'huile présente une taille primaire moyenne en nombre comprise entre 1 et 100 µm, de préférence comprise entre 5 et 30 µm.

26. Composition selon l'une quelconque des revendications précédentes, **caractérisée en ce que** les tensioactifs anioniques sont choisis parmi les sels de métaux alcalins, les sels d'ammonium, les sels d'amines, les sels d'aminoalcools ou les sels de métaux alcalino-terreux, des composés suivants : les sulfates d'alkyle, les alkyléther-sulfates, les alkylamidoéthersulfates, les alkylarylpolyéthersulfates, les monoglycéride-sulfates ; les alkylsulfonates, les phosphates d'alkyle, les alkylamidesulfonates, les alkylarylsulfonates, les alpha-oléfine-sulfonates, les paraffine-sulfonates ; les sulfosuccinates d'alkyle, les alkyléthersulfosuccinates, les alkylamidesulfosuccinates ; les sulfoacétates d'alkyle ; les acylsarcosinates ; et les acylglutamates, les groupes alkyle ou acyle de tous ces composés comportant de 6 à 24 atomes de carbone et le groupe aryle désignant de préférence un groupe phényle ou benzyle ; les esters d'alkyle en C₆-C₂₄ et d'acides polyglycoside-carboxyliques ; les sulfosuccinamates d'alkyle, les iséthionates d'acyle et les N-acyltaurates, le groupe alkyle ou acyle de tous ces composés comportant de 12 à 20 atomes de carbone.

27. Composition selon la revendication 26, **caractérisée en ce que** les tensioactifs anioniques sont choisis parmi les sels de sulfates d'alkyle, d'alkyléther-sulfates, de préférence à 2 ou 3 moles d'oxyde d'éthylène, et d'alkyléther-carboxylates, les groupes alkyle comportant de 6 à 24 atomes de carbone.

28. Composition selon l'une quelconque des revendications 1 à 27, **caractérisée en ce que** les tensioactifs non ioniques sont choisis parmi les alcools, les alpha-diols, les alkyl(C₁-C₂₀)phénols ou les acides gras polyéthoxylés, polypropoxylés ou polyglycérolés, ayant une chaîne grasse, le nombre de groupements oxyde d'éthylène ou oxyde de propylène pouvant aller notamment de 2 à 50 et le nombre de groupements glycérol pouvant aller de 2 à 30 ; les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines ou les oxydes de N-acyl(C₁₀-C₁₄)aminopropylmorpholine.

29. Composition selon la revendication 28, **caractérisée en ce que** les tensioactifs non ioniques sont choisis parmi les alkyl(C₆-C₂₄)polyglycosides et plus particulièrement les alkyl(C₈-C₁₆)polyglycosides.

30. Composition selon l'une quelconque des revendications précédentes, **caractérisée en ce que** les tensioactifs anioniques et/ou non ioniques sont présents en une quantité comprise entre 3 et 50 % en poids, de préférence entre 4 et 30 % en poids par rapport au poids total de la composition.

31. Composition selon l'une quelconque des revendications 1 et 2 à 30, **caractérisée en ce qu'**elle comprend en outre un ou plusieurs tensioactifs amphotères.

32. Composition selon l'une des revendications 2 à 31, **caractérisée en ce que** les tensioactifs amphotères sont choisis parmi les alkyl(C₈-C₂₀)bétaïnes, les alkyl(C₈-C₂₀)amidoalkyl(C₆-C₈)bétaïnes, les alkylamphodiacétates.

33. Composition selon l'une quelconque des revendications 2 à 32, **caractérisée en ce que** les tensioactifs amphotères sont présents en une quantité comprise entre 0,1 et 20 % en poids, de préférence entre 0,5 et 15 % en poids par rapport au poids total de la composition.

34. Composition selon l'une quelconque des revendications précédentes, **caractérisée en ce que** le milieu cosmétiquement acceptable comprend l'eau ou un mélange d'eau et d'un solvant cosmétiquement acceptable.

35. Composition selon la revendication 34, **caractérisée en ce que** le solvant cosmétiquement acceptable est choisi parmi les alcools inférieurs en C₁-C₄, les polyols et leurs mélanges.

36. Composition selon l'une quelconque des revendications précédentes, **caractérisée en ce qu'**elle comprend en outre un ou plusieurs additifs classiques choisis parmi des agents tensioactifs cationiques, des polymères anioniques, cationiques, non-ioniques amphotères ou zwitteroniques, des filtres UV, des parfums, des colorants, des épaississants naturels ou synthétiques, des alcools gras en C₁₂-C₃₀, des agents nacrants, des conservateurs, des agents de stabilisation de pH, des vitamines, des provitamines, des agents antimicrobiens, des agents anti-pélliculaires ou anti-séborrhéiques, les agents anti-oxydants ou réducteurs, et les agents acides ou alcalins.

37. Composition selon l'une quelconque des revendications précédentes, **caractérisée en ce que** la composition comprend en outre au moins un polymère cationique.

38. Composition selon la revendication 37, **caractérisée en ce que** les polymères cationiques sont choisis parmi les dérivés d'éther de cellulose quaternaires, les gommes de guar cationiques, les cyclopolymères cationiques, les polymères quaternaires de vinylpyrrolidone et de vinylimidazole, et leurs mélanges.

39. Composition selon l'une des revendications 36 à 38, **caractérisée en ce que** le ou les polymères cationiques sont présents à des concentrations allant de 0,01 à 20%, de préférence de 0,05 à 10% et plus particulièrement de 0,1 à 5 % en poids par rapport au poids total de la composition.

40. Composition selon l'une quelconque des revendications précédentes, **caractérisée en ce qu'**elle se présente sous la forme d'une émulsion huile-dans-eau.

41. Utilisation de la composition selon l'une quelconque des revendications précédentes, comme shampoing.

42. Procédé de traitement des cheveux consistant à appliquer sur les cheveux, une quantité efficace d'une composition telle que décrite à l'une quelconque des revendications 1 à 39, et à rincer après un éventuel temps de pose.
